# EUROPÄISCHE PATENTSCHRIFT

(11) **EP 0 523 446 B1**
(45) Veröffentlichungstag und Bekanntmachung des Hinweises auf die Patenterteilung: **04.12.1996**
(21) Anmeldenummer: 92111104.3
(22) Anmeldetag: 01.07.1992
(51) Int. Cl.: G01N 33/38

(54) **Verfahren und Vorrichtung zur laufenden Kontrolle des Gehaltes von Chloriden in Rauchgasgips**
Method and apparatus for continuously monitoring the content of chlorides in flue gas gypsum
Procédé et dispositif de contrôle de la teneur en chlorures de plâtre de gaz de fumée

(30) Priorität: 17.07.1991 DE 4123736
(43) Veröffentlichungstag der Anmeldung: 20.01.1993
(73) Patentinhaber: Gebr. Knauf Westdeutsche Gipswerke KG, D-97343 Iphofen (DE)
(72) Erfinder: Hüller, Rolf. Dr., W-8712 Volkach (DE); Wirsching, Franz, Dr., W-8715 Iphofen (DE)
(74) Vertreter: Werner, Hans-Karsten, Dr.Dipl.-Chem.

(56) Entgegenhaltungen:
- EP-A- 0 397 235
- DE-A- 3 822 847
- GB-A- 2 226 402

## Beschreibung

Gegenstand der vorliegenden Erfindung ist ein verbessertes Verfahren und eine Vorrichtung zur laufenden Kontrolle des Gehaltes von wasserlöslichen Chloriden in Rauchgasgips durch Probenahme, Probevorbereitung, Messung und Auswertung.

Gattungsgemäße Verfahren sind bekannt aus der DE-OS 38 22 847 und der DE-OS 38 43 240. Die dort beschriebenen Verfahren haben sich prinzipiell bewährt. Es bestand jedoch das Bedürfnis, diese Verfahren und Vorrichtungen weiter zu verbessern, zu verkleinern, dabei jedoch robuster und noch weniger störanfällig zu machen unter den Bedingungen eines technischen Betriebes. Insbesondere bestand die Aufgabe darin, die zu analysierende repräsentative Probe zu verkleinern, einwandfrei zu suspendieren und in der wäßrigen Phase einwandfrei den Chloridgehalt automatisch zu bestimmen, wobei das Mischgefäß zwischen zwei Suspendierungen einfach, preiswert und zuverlässig gereinigt werden sollte. Dabei sollte der Verbrauch an entmineralisiertem Wasser möglichst gering gehalten werden.

Diese Aufgabe konnte jetzt überraschend einfach dadurch gelöst werden, daß
a) mit Hilfe eines kombinierten Probenehmers und Probeteilers eine repräsentative Probe des von einer Abwurfschurre herabfallenden Rauchgasgipses entnommen wird,
b) diese repräsentative Probe in eine zuvor vorgelegte und gewogene Menge Leitungswasser oder Trinkwasser eingebracht und durch einen Rührer suspendiert wird,
c) diese Suspension nach Abstellen des Rührers während oder nach der Sedimentation gewogen wird,
d) nach der Sedimentation eine Teilmenge des Überstandes entnommen und mit Salpetersaüre angesaüert wird,
e) in dieser Teilmenge durch automatisierte potentiometrische Titration mit einer filbernitratlösung gegen eine Silberelektrode titriert und somit der Chloridgehalt bestimmt wird,
wobei der auf die eingewogene Probemenge rückbezogene Meßwert für Chlorid automatisch registriert wird und gewünschtenfalls bei Überschreitung eines vorgegebenen Grenzwertes ein Signal auslöst und/oder den Transport des zu chloridreichen Rauchgasgipses zur Hauptmenge unterbindet.

Die verbesserte Vorrichtung zur Durchführung des Verfahrens besteht aus
a) einem mit konstanter Geschwindigkeit durch einen von einer Abwurfschurre herabfallenden Rauchgasgipsstrom hindurchfahrenden Löffel mit verstellbarem Eintrittsschlitz und in der Ausgangsstellung mechanisch betätigter Entleerungsklappe zwecks Entnahme von 100 bis 1.500 g repräsentativer Probe,
b) einer Fördervorrichtung zur Beförderung der repräsentativen Probe zu einem oben offenen zylindrokonischen Mischgefäß mit einem Fassungsvermögen von mindestens 5 l, vorzugsweise 8 bis 20 l, wobei dieses Mischgefäß ausgerüstet ist mit einem automatisch an- und abschaltbaren Rührer, einer Zuleitung für automatisch dosierbare Wassermengen, eine in den oberen Teil des Mischgefäßes hereinragende Absaugleitung für die Teilmenge des Überstandes sowie einer robusten automatischen Waage,
c) einem Titrationsautomaten für die potentiometrische Titration von Chlorid mit Silbernitrat gegen eine Silberelektrode mit automatischer Bestimmung, Registrierung und gegebenenfalls Steuerung der Anlage und/oder einem Signalauslöser, der auch die weitere Ausschleusung des zu chloridreichen Rauchgasgipses unterbindet, sowie einem Rechen- und Steuerungsgerät für die gesamte Anlage.

Es ist erfindungsgemäß möglich, die Menge der repräsentativen Probe von mindestens 5.000 g auf 100 bis 1.500 g zu reduzieren. Vorzugsweise wird mit Proben von 300 bis 600 g Rauchgasgips gearbeitet. Das zylindrokonische Mischgefäß kann erfindungsgemäß so verkleinert werden, daß es nur noch mindestens 5 l, vorzugsweise 8 bis 20 l faßt. Bei einer vorgelegten Wassermenge von ca. 8 l und einer repräsentativen Probe von Rauchgasgips zwischen 300 und 600 g genügt ein Mischgefäß mit einem Fassungsvermögen von 10 l.

Um Störungen durch Verbackungen völlig zu vermeiden, wird erfindungsgemäß das Wasser vorgelegt und in dieses die repräsentative Probe unter Rühren eingebracht und suspendiert. Es werden mit Hilfe der automatischen Waage sowohl die vorgelegte Wassermenge als auch die Gesamtsuspension gewogen. Das Wiegen erfolgt jeweils bei abgeschaltetem Rührer, wodurch sich die Genauigkeit der Wägung erhöht. Die Wägung der Suspension kann erfindungsgemäß erfolgen während oder nach der Sedimentation.

Erfindungsgemäß wird bereits nach teilweiser Sedimentation eine Teilmenge des Überstandes entnommen und direkt in den Titrationsautomaten überführt. Das Mischgefäß wird vorzugsweise unter Wiedereinschaltung des Rührers entleert und anschließend mit frischem Wasser gespült. Hierfür hat sich ein berührungsfrei in das Mischgefäß hereinragender Ring mit automatisch ein- und ausschaltbaren Sprühdüsen für Wasser bewährt. Das zur Suspendierung notwendige Wasser wird hingegen über eine separate Zuleitung vorgelegt, die mit einer Mengendosiereinheit verbunden ist. Diese Trennung der Wasserzuführungen gestattet es, das Spülen des Mischgefäßes mit normalem Leitungs- oder Trinkwasser durchzuführen. Darüber hinaus kann auch die gesamte Analytik mit Leitungs- oder Trinkwasser durchgeführt werden, sofern dessen Chloridgehalt von Zeit zu Zeit bestimmt und von dem gemessenen Chloridgehalt des Rauchgasgipses abgezogen wird. Der teilweise oder vollständige Verzicht auf die Verwendung von entmineralisiertem Wasser senkt die laufenden Kosten der Analytik erheblich.

Für die eigentliche Chloridbestimmung hat sich die automatisierte potentiometrische Titration mit Silbernitrat gegen eine Silberelektrode besonders bewährt. Bei dieser Bestimmung ist es nicht nötig, den Überstand zu filtrieren oder sonst vollständig von den verbliebenen Schwebstoffen zu befreien. Es entfällt somit auch der Einsatz eines Beipaßfilters, einer Beipaßzentrifuge und deren Pflege und Reinigung. Es genügt, wenn die zu bestimmende Probe mit Salpetersäure angesäuert wird.

Ein weiterer Vorteil des erfindungsgemäßen Verfahrens besteht darin, daß die Mengen an Suspension und titrierten überstand so gering sind, daß sie problemlos dem Abwasser der Rauchgasentschwefelungsanlage zugeführt werden können. Es kann somit auf spezielle Rückführungsleitungen zur Rauchgasentschwefelungsanlage verzichtet werden.

Die Meßergebnisse und Regel impulse aus den Chloridwerten liegen beim erfindungsgemäßen Verfahren nach insgesamt 8 bis 12 Minuten vor. Diese Verzögerung kann erfahrungsgemäß in Kauf genommen werden. Es sind sogar die Vorlaufmengen und Nachlaufmengen beim Ausschleusen von Rauchgasgips so gering, daß der erhöhte Wassergehalt im Vorlauf und ein etwaig leicht erhöhter Chloridgehalt im Vorlauf oder Nachlauf, bezogen auf die Gesamtmenge des Rauchgasgipses, unbeachtlich und nicht störend sind.

Als kombinierter Probenehmer und Probeteiler hat sich vor allem ein mit konstanter Geschwindigkeit durch ein mit einer Abwurfschurre herabfallenden Rauchgasgipsstrom hindurchfahrbarer Löffel mit verstellbarem Eintrittsschlitz und in der Ausgangsstellung mechanisch betätigter Entleerungsklappe bewährt. Derartige Geräte sind beispielsweise von der Glasindustrie für die Probenahme rieselfähiger Komponenten eingesetzt worden. Es wurde jetzt gefunden, daß auch der zum Verkleben neigende feuchte Rauchgasgips mit diesen Geräten einwandfrei als repräsentative Probe entnommen werden kann. Voraussetzung hierfür ist allein, daß der Rauchgasgips über eine Abwurfschurre abgeworfen wird und der Löffel durch diesen herabfallenden Strom des Rauchgasgipses hindurchgeführt wird. Bewährt hat sich insbesondere der Probenehmer Typ LA der Retsch-Anlagentechnik GmbH, Haan.

Die von diesem Gerät entnommene repräsentative Probe wird erfindungsgemäß mit einer Fördervorrichtung, beispielsweise einer gegebenenfalls mit einem Klopfer versehenen Rutsche oder mit einem Förderband zu dem oben offenen zylindrokonischen Mischgefäß befördert, wo die Probemenge in eine bereits vorgelegte Wassermenge eingerührt und suspendiert wird. Das Mischgefäß ist mit einer automatischen Waage fest verbunden. Der Rührer, die Zuleitung für vorzulegendes Wasser sowie der Ring mit Sprühdüsen zum Reinigen des Gefäßes ragen berührungslos in dieses Gefäß hinein. Etwa in das obere Viertel des Gefäßes ragt die Absaugleitung für den Überstand hinein, über die die Teilmenge des Überstands zum Titrationsautomaten geleitet wird. Im Titrationsautomaten wird die Probe mit Salpetersäure angesäuert und danach mit Silbernitrat gegen eine Silberelektrode titriert. Das Meßergebnis wird registriert und auf die eingewogene Menge der Suspension rückbezogen ermittelt. Kontrollmessungen des Chloridgehalts im Rauchgasgips nach der klassischen analytischen Methode haben ergeben, daß die erfindungsgemäß ermittelten Chloridwerte sehr gut korrelieren und daher ausgezeichnet geeignet sind, die Gesamtanlage zu regeln und zu steuern.

Als Titrationsautomat bewährt hat sich insbesondere der Titrolyzer ADI 2011, der von der Firma Deutsche Metrohm GmbH & Co., Filderstadt, angeboten und vertrieben wird.

Weiterhin besteht die erfindungsgemäße Vorrichtung aus einer automatischen Steuerung, beispielsweise der Simatic S5/100U und einem handelsüblichen Drucker. Diese Steuerung weist den Vorteil auf, vollautomatisch zu laufen, jedoch in einfachster Weise auf Handbetrieb umstellbar zu sein.

In der anliegenden Schemazeichnung ist die erfindungsgemäße Vorrichtung näher erläutert. Darin stellen dar:
Die Gruppe 1 die Probeentnahme,
die Gruppe 2 die Analysenaufbereitung,
die Gruppe 3 die elektronische Waage,
die Gruppe 4 den Titrationsautomaten und
die Gruppe 5 die Steuerung.

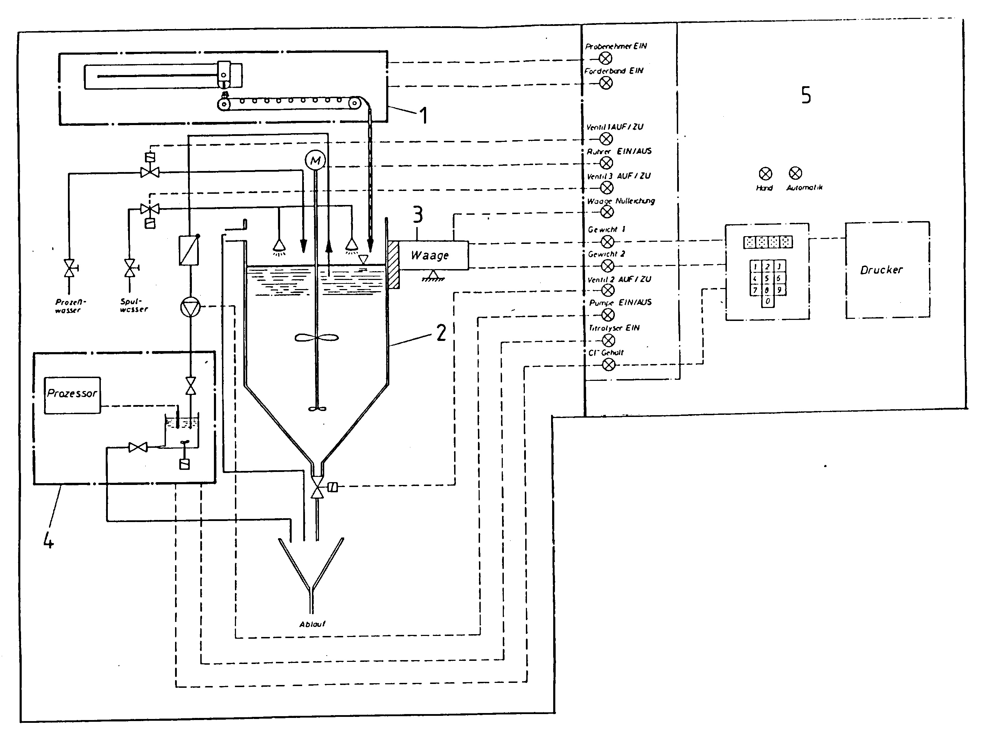

## Patentansprüche

1. Verfahren zur laufenden Kontrolle des Gehaltes von wasserlöslichen Chloriden in Rauchgasgips durch Probenahme, Probevorbereitung, Messung und Auswertung, dadurch gekennzeichnet, daß
a) mit Hilfe eines kombinierten Probenehmers und Probeteilers eine repräsentative Probe des von einer Abwurfschurre herabfallenden Rauchgasgipses entnommen wird,
b) diese repräsentative Probe in eine zuvor vorgelegte und gewogene Menge Leitungswasser oder Trinkwasser eingebracht und durch einen Rührer suspendiert wird,
c) diese Suspension nach Abstellen des Rührers während oder nach der Sedimentation gewogen wird,
d) nach der Sedimentation eine Teilmenge des Überstandes entnommen und mit Salpetersäure angesäuert wird,
e) in dieser Teilmenge durch automatisierte potentiometrische Titration mit einer Silbernitratlösung gegen eine Silberelektrode titriert und somit der Chloridgehalt bestimmt wird,
wobei der auf die eingewogene Probemenge rückbezogene Meßwert für Chlorid automatisch registriert wird und bei Überschreitung eines vorgegebenen Grenzwertes ein Signal auslöst und/oder den Transport des zu chloridreichen Rauchgasgipses zur Hauptmenge unterbindet.

2. Verfahren gemäß Anspruch 1, dadurch gekennzeichnet, daß die repräsentative Probe 100 bis 1.500 g beträgt.

3. Verfahren gemäß Anspruch 1 oder 2, dadurch gekennzeichnet, daß die vorgelegte Wassermenge 8 bis 20 1 beträgt.

4. Verfahren gemäß einem der Ansprüche 1 bis 3, dadurch gekennzeichnet, daß der Chloridgehalt des Wassers von Zeit zu Zeit bestimmt und bei der Berechnung des Chloridgehalts des Rauchgasgipses subtrahiert wird.

5. Vorrichtung zur Durchführung des Verfahrens gemäß Ansprüchen 1 bis 4 bestehend aus
a) einem mit konstanter Geschwindigkeit durch einen von einer Abwurfschurre herabfallenden Rauchgasgipsstrom hindurchfahrenden Löffel mit verstellbarem Eintrittsschlitz und in der Ausgangsstellung mechanisch betätigter Entleerungsklappe zwecks Entnahme von 100 bis 1.500 g repräsentativer Probe,
b) einer Fördervorrichtung zur Beförderung der repräsentativen Probe zu einem oben offenen zylindrokonischen Mischgefäß mit einem Fassungsvermögen von mindestens 5 l, vorzugsweise 8 bis 20 l, wobei dieses Mischgefäß ausgerüstet ist mit einem automatisch an- und abschaltbaren Rührer, einer Zuleitung für automatisch dosierbare Wassermengen, eine in den oberen Teil des Mischgefäßes hereinragende Absaugleitung für die Teilmenge des Überstandes sowie einer robusten automatischen Waage,
c) einem Titrationsautomaten für die potentiometrische Titration von Chlorid mit Silbernitrat gegen eine Silberelektrode mit automatischer Bestimmung, Registrierung und Steuerung der Anlage und einem Signalauslöser, der auch die weitere Ausschleusung des zu chloridreichen Rauchgasgipses unterbindet, sowie einem Rechen- und Steuerungsgerät für die gesamte Anlage.

6. Vorrichtung gemäß Anspruch 5, dadurch gekennzeichnet, daß in das Mischgefäß ein Ring mit Sprühdüsen montiert ist durch welche zwischen zwei Suspendierungen das Mischgefäß mit Hilfe von Spülwasser gereinigt wird.

## Claims

1. A method for continuously monitoring the content of watersoluble chlorides in flue gas gypsum by sampling, sample preparation, measurement and evaluation, characterized in that
a) a representative sample of said flue gas gypsum dropping from a discharge chute is collected by means of a combined sampler and sample divider;
b) said representative sample is introduced in a previously provided and weighed quantity of tap water or drinking water and suspended with a stirrer;
c) the suspension is weighed, after turning off the stirrer, during or after the sedimentation;
d) a fraction of the supernatant is removed after the sedimentation and acidified with nitric acid;
e) titration is performed with this fraction by automated potentiometric titration with a silver nitrate solution against a silver electrode, thus determining the chloride content;
wherein the measured value for chloride, recalculated for the sample quantity weighed, is automatically registered and, when a predetermined limit is exceeded, a signal is triggered and/or the transport of the flue gas gypsum which is too rich in chloride to the bulk is stopped.

2. The method according to claim 1, characterized in that the mass of said representative sample is from 100 to 1,500 g.

3. The method according to claim 1 or 2, characterized in that said previously provided quantity of water is from 8 to 20 l.

4. The method according to any of claims 1 to 3, characterized in that the chloride content of said water is determined from time to time and subtracted in the calculation of the chloride content of said flue gas gypsum.

5. A device for performing the method according to claims 1 to 4, consisting of
a) a bucket having an adjustable inlet port and a discharge door which is mechanically operated in its initial position which bucket moves at a constant velocity through a current of flue gas gypsum dropping from a discharge chute for the purpose of collecting from 100 to 1,500 g of representative sample;
b) a conveying device for conveying said representative sample to an open-top cylindro-conical mixing vessel having a capacity of at least 5 l, preferably from 8 to 20 l, said mixing vessel being equipped with a stirrer which is automatically turned on and off, a feeding line for automatically metered quantities of water, a suction line protruding in the upper portion of said mixing vessel for said fraction of supernatant, and a robust automatic scale;
c) an automatic titration apparatus for potentiometric titration of chloride with silver nitrate against a silver electrode involving automated determination, registration and control of the installation and having a signal trigger which also stops further outward transfer of the flue gas gypsum which is too rich in chloride, as well as a computing and controlling device for the entire installation.

6. The device according to claim 5, characterized in that a ring having spraying nozzles is mounted in said mixing vessel by which said mixing vessel is cleansed with rinsing water between two suspending operations.

## Revendications

1. Procédé pour le contrôle continu de la teneur en chlorures hydrosolubles d'un plâtre provenant de la désulfuration des gaz de fumée, par prélèvement d'échantillons, préparation des échantillons, mesure et évaluation des résultats, caractérisé en ce que :
a) à l'aide d'un dispositif de prélèvement d'échantillons et d'un diviseur d'échantillons, combinés, on prélève un échantillon représentatif du plâtre de désulfuration des gaz de fumée tombant d'une goulotte de distribution,
b) on introduit cet échantillon représentatif dans une quantité d'eau du robinet ou d'eau potable préalablement introduite et pesée et on le met en suspension dans cette eau au moyen d'un agitateur,
c) après avoir stoppé l'agitateur, on pèse cette suspension, pendant ou après la sédimentation,
d) après la sédimentation, on prélève une quantité partielle du surnageant et on l'acidifie avec de l'acide nitrique,
e) on effectue un titrage dans cette quantité partielle, par potentiométrie automatique, avec une solution de nitrate d'argent contre une électrode en argent, et on détermine ainsi la teneur en chlorures,
la valeur mesurée pour le chlorure, rapportée à la quantité pesée d'échantillon, étant enregistrée automatiquement et déclenchant un signal et/ou empêchant le transport du plâtre de désulfuration des gaz de fumée trop riche en chlorures vers la quantité principale quand une valeur limite prédéfinie est dépassée.

2. Procédé selon la revendication 1, caractérisé en ce que l'échantillon représentatif est de 100 à 1 500 g.

3. Procédé selon la revendication 1 ou 2, caractérisé en ce que la quantité d'eau préalablement introduite est de 8 à 20 l.

4. Procédé selon l'une des revendications 1 à 3, caractérisé en ce que la teneur en chlorures de l'eau est déterminée de temps à autre et soustraite pour le calcul de la teneur en chlorures du plâtre de désulfuration des gaz de fumée.

5. Dispositif pour la mise en oeuvre du procédé selon les revendications 1 à 4, comprenant :
a) une cuillère traversant, à vitesse constante, un flux de plâtre de désulfuration des gaz de fumée tombant d'une goulotte de distribution, comportant une fente d'entrée réglable, et, à la sortie, un clapet d'évacuation actionné mécaniquement pour prélever de 100 à 1 500 g d'échantillon représentatif,
b) un dispositif d'acheminement pour amener l'échantillon représentatif dans un récipient mélangeur cylindro-conique ouvert vers le haut, d'une contenance d'au moins 5 l, de préférence de 8 à 20 l, ce récipient mélangeur étant équipé d'un agitateur pouvant être actionné et stoppé automatiquement, d'une canalisation d'amenée pour des quantités d'eau pouvant être dosées automatiquement, d'une canalisation d'aspiration pénétrant dans la partie haute du récipient mélangeur pour la quantité partielle du surnageant, ainsi que d'une balance automatique robuste,
c) un appareil automatique d'analyse volumétrique pour la potentiométrie du chlorure avec du nitrate d'argent contre une électrode en argent, avec détermination, enregistrement et commande automatiques de l'installation et avec un déclencheur de signaux qui empêche aussi la poursuite de l'évacuation du plâtre de désulfuration des gaz de fumée trop riche en chlorures, ainsi qu'un appareil de calcul et de commande pour l'ensemble de l'installation.

6. Dispositif selon la revendication 5, caractérisé en ce qu'un anneau est monté dans le récipient mélangeur, comportant des tuyères de pulvérisation au moyen desquelles le récipient mélangeur est nettoyé à l'aide d'eau de rinçage entre deux mises en suspension.
